# EUROPEAN PATENT SPECIFICATION

(11) **EP 1 549 272 B1**
(45) Date of publication and mention of the grant of the patent: **13.04.2011**
(21) Application number: 03736415.5
(22) Date of filing: 30.06.2003
(51) Int. Cl.: A61F 13/496

(54) **ABSORBENT PRODUCT FOR MEN**
SAUGFÄHIGES PRODUKT FÜR MÄNNER
PRODUIT ABSORBANT POUR HOMMES

(30) Priority: 05.07.2002 SE 0202101
(43) Date of publication of application: 06.07.2005
(73) Proprietor: SCA Hygiene Products AB, 405 03 Göteborg (SE)
(72) Inventor: STRANNEMALM, Kenneth, S-448 31 Floda (SE)
(74) Representative: Olsson, Stefan
(86) International application number: PCT/SE2003/001138
(87) International publication number: WO 2004/004619

(56) References cited:
- WO-A1-96/05786
- US-A- 4 326 302
- US-A- 4 589 877
- US-A- 4 944 733
- US-A- 5 290 270
- US-A- 5 843 065

## Description

### TECHNICAL FIELD

The present invention relates to a pants-shaped absorbent product, such as incontinence pants, intended for males, comprising a front section, a rear section, a crotch section, side sections, which are intended to connect the front section and the rear section in the lateral direction and which comprise hip sections, an elastic waist region and an absorbent element which, when the article is in use, is intended to at least cover the penis of the user, and further comprising a liquid-tight outer layer, which is intended to enclose the absorbent element at least on the side thereof facing away from the user when the product is worn.

### BACKGROUND ART

Absorbent disposable products for absorbing urine have developed a lot since entering into more general use during the 60's and 70's. Since they are disposable products, it is necessary for them to be able to be produced and sold at a very low price. At the same time, it is important that the functioning of the products should be good and reliable. Good fit and comfort are also important characteristics. The first disposable nappies were constituted by two-piece products, outer pants made of plastic, which were intended for reuse, and a rectangular absorbent insert article for single usage. The absorbent material in these insert articles was initially constituted by cellulose. Later better absorption material, consisting of so-called cellulose fluff, was developed. The fit and comfort of these early nappies was poor. The products were cumbersome and uncomfortable for the user. In the late 70's, the first so-called complete disposable nappies were born, i.e. nappies in which the absorption bodies were integrated with a liquid-tight outer layer. The absorption materials were developed and became better, which meant that the absorption bodies could be better adapted to the body shape of the user. Hourglass-shaped absorption cores having a narrower crotch section between the two end sections are now dominant. The trend has also been towards thinner and thinner products, which has been made possible by the mixing of so-called superabsorbent material into the absorption body. There are numerous reasons why thinner and also smaller absorption bodies are being sought. A thinner and smaller absorption body is more comfortable and more discrete, which, not least, is important for adult incontinent users. A reduction in volume is also economically very important, since less storage space is needed, transportation is made easier and fewer metres of shelving are needed in the shops. The last-named is important for shop economics and if products can be developed which require less shop space than competitors' products, a not inconsiderable competitive advantage is gained. Moreover, there is increased pressure from the authorities to use as little material as possible, especially in disposable articles, with a view to lessening environmental pollution.

The smaller the absorption bodies, the more important is it that the absorption bodies should land in the right place directly in front of the genitals of the user and should remain in place during use, even when the user is very active and moving a lot. Consumers are also becoming increasingly demanding about discretion and comfort and also reliability. The requirements that the absorption body will end up correctly positioned when it is donned and will thereafter be held in the right place have therefore increased the need for the product to be securely fixed on the body and for it to respond closely to the body when the user moves, at the same time as the requirements have increased for the product always to land in the right place when the article is applied to the user. This has led to the development of so-called pull-ups, which have elastic sections for enhanced fit and comfort and enhanced responsiveness to the movements of the user compared with conventional absorbent products.

An early patent publication relating to disposable-type pants-shaped absorbent product is GB 2 112 267-A. This publication from 1983 shows, however, primitive pants, which never became a commercial product. Not until the 1990's did absorbent products having a pants-like shape and design become a commercial success. Pants-like products now exist in the form of children's nappies and adults' nappies.

Pants-shaped absorbent products which have hitherto been commercially available have been configured, in principle, like conventional nappies, having a front section and a rear section and an intermediate crotch section, the front and rear section being mutually joined.

Known pants-shaped absorbent products which are available on the market work satisfactorily for women. As far as male incontinence is concerned, there are so far no pants-shaped product which are satisfactory in every respect.

A major problem for male incontinence sufferers who do not have any movement-related problems is that public toilets for men have urinals and that the use of these requires the user to have pants with a fly. It is naturally inconceivable for a man, in connection with a toilet visit at a urinal, to pull down the whole of the pants-shaped product and thereby reveal private and embarrassing problems to those around him. The consequence of this is that even relatively minor incontinence problems become very embarrassing for men in everyday life. Visits to sports arenas and other major events are, in practice, almost impossible for men with incontinence problems. Correspondingly, so-called "training pants", which are used when children have to be weaned off nappies, also to some extent represent a problem for small boys. Over the years, there have been a host of solutions proposed to the abovementioned problems. Common to all these proposed solutions is that the nappy or training pants has been provided with some form of opening, which is intended to serve as a fly opening.

In US patent 4 326 302, a two-piece product is described having pants provided with a pocket for receiving an absorption element, which, when the pants are used, is situated over the genital region of the user. The pants are provided with an opening next to the pocket, which opening is intended to serve as a fly opening. This solution does not work satisfactorily, since the absorption element has to be firmly displaced in the lateral direction in order for the user to get the opportunity to fulfil his needs via the opening. In addition, it is awkward and time-consuming to bring the absorption element into the correct position following use of the opening.

In US patent 4 589 877 an insert is described in which the absorption element is divided and in which the parts are fitted in overlapping arrangement with a fly opening between the parts. A problem with this design is that the insert in the region of the overlap is bulky and cumbersome. A product according to this publication is, moreover, difficult to produce. There is also a risk of leakage through the fly opening.

In US patent 4 944 733 a nappy is described which has been provided with an opening extending from the waist region to the crotch region of the nappy. The opening is constituted by an elongated slot and this opening is closed by sections on both sides of the opening being fitted in overlapping arrangement. The nappy is provided with adhesive fasteners which hold the opening in closed position. A fundamental drawback with this nappy design is the said adhesive fasteners, which are subjected to high stresses when the nappy is worn and when the fasteners have to be undone to free the opening. Production of products according to the last-named patent is troublesome and costly.

In US patent 5 843 065 an incontinence nappy for men is described, which nappy is provided with an opening situated directly in front of the penis of the user and through which an absorption pad situated inside the opening can be folded out for access to the penis inside the slot. This design is impractical in practice, especially when the absorption pad has to be returned to its intended place inside the opening following a toilet visit. An incontinence nappy according to US patent 5 843 065 is, moreover, both awkward and expensive to produce.

In US patent 5 290 270 a men's undergarment is disclosed.

No satisfactory solution to the problem has hitherto been presented and there are presently no products whatsoever on the market despite the fact that the problem is old and well known.

### DISCLOSURE OF INVENTION

As a result of the present invention, an improved pants-shaped absorbent product has been produced, which product wholly eliminates the abovementioned problems.

A product of the type stated in the introduction is characterized according to the invention in that the front section has at least one elastic member, which, during product usage, enables the front section of the product to be pulled down, counter to the action of the said elastic member, to a position in which the upper limit edge of the front section in the middle region of the front section is situated below the penis of the user, at the same time as at least the hip sections of the product are arranged to be held in place around the waist of the user, in that the absorbent element is configured with one or more deformation zones, which enable those parts of the absorbent element which are situated above and over the penis of the user during product usage to be drawn down together with the rest of the front section when the front section of the product is pulled down, and in that the front section of the product, together with the absorbent element, are arranged to be returned by the elastic member to their original usage position.

In the product according to the present invention, no special fly opening is therefore needed on the absorbent product, but rather a user introduces his hand through the fly opening in the outer pants and, by a simple manoeuvre, guides down the front section of the absorbent product in order to free the penis.

According to one embodiment, the invention is characterized in that the said elastic member, over the whole of its working range, has an essentially constant tensile stress when stretched, which tensile stress is in the same order of magnitude as the tensile stress in the elastic waist region.

According to one embodiment, the invention is characterized in that the elastic member is constituted by a section of the elastic waist region. According to one embodiment, the said section extends over the front section between the said hip sections.

According to one embodiment, the product according to the invention is characterized in that the elastic waist region extends over the whole of the front section, rear section and side sections of the product and in that the said section of the elastic waist region which serves as the elastic member has higher elastic stretchability than the rest of the elastic waist region.

According to a further embodiment, the invention is characterized in that the absorbent element is of thinner and more flexible configuration in the deformable zone compared with the rest of the absorbent element.

According to another embodiment, the invention is characterized in that the absorbent element incorporates deformable zones in the form of fold notches for the absorbent element, along which fold notches the product is bent when the front section of the product is pulled down.

According to a further embodiment, the invention is characterized in that the said fold notches are constituted by recesses, through-slots or compression lines in the absorbent element.

According to another embodiment, the product according to the invention is characterized in that the absorbent element is constituted by at least two absorption bodies of different extent, in that only one of these extends into the deformation zone and in that the absorption body extending into the deformation zone has little or no inherent stiffness.

According to another embodiment, the product according to the invention is characterized in that the front section of the product, at the waist region, has a viewed from the crotch region - concave recess between the said hip sections, whereby the front section of the product, during product usage, acquires a low pants cut, which, compared with a straight cut, reduces the amount by which the upper limit edge of the product in the middle region of the front section needs to be pulled down in order to bring the said limit edge into position below the penis of the user.

According to one embodiment, the product according to the invention is characterized in that the hip sections of the product are provided with friction members, which are arranged to help the product as a whole to be held in place on the user as the front section of the product is pulled down.

According to a further embodiment, the product according to the invention is characterized in that the said elastic waist section is formed from an elastic first piece which, in the extended state, is essentially rectangular and which is intended to partially surround the trunk of the user and form the rear section and side sections of the pants-like product, in that a second piece, incorporated in the product, is configured to form the front section and crotch section of the pants-like product, in that the said second piece is elongated with two opposing end edges and two opposing longitudinal edges, in that the width of the second piece, at least at the crotch section, is less than the length of the first piece, in that the second piece with its longitudinal direction is arranged perpendicularly to the longitudinal direction of the first piece and the width of the second piece, at least at the crotch section, is less than the length of the first piece, in that the second piece with its longitudinal direction is arranged perpendicularly to the longitudinal direction of the first piece and is connected by a first end section to the one longitudinal edge section of the first piece and centrally on this, in that the one end section of the first piece is connected to a first side edge section of the second piece, and in that the second end section of the first piece is correspondingly connected to a second side edge section of the second piece, and the absorbent element is fitted, in its entirety, on the second piece.

By virtue of the fact that also the whole of the rear section is elastic and, together with the elastic side sections, forms a single elastic continuous first piece, the pants as a whole are very responsive to body movements. Local disturbances are absorbed and smoothed out by the continuous elastic piece and are not transmitted to the less resilient parts of the front section and crotch section situated directly in front of the genitals of the user. Compared with conventional nappies and previously known pants-shaped absorbent products, with this embodiment of the product according to the invention a superior fit and comfort are obtained. The greater part of the pants-like garment remains totally plain. The seams which are needed are fitted at the transition between the front section and the first piece and at the transition between the first and second piece at the crotch section. These seams end up in places which are not subjected to any great pressure during product usage and there is less risk of chafing and pressure sores caused by seams on the pants-like product compared with known pants-shaped products currently available.

The configuration of the pants sheath from only two part-pieces and in which the absorbent element is situated entirely on the second part-piece offers increased freedom in the choice of production method compared with previously known pants-shaped products. inner side of an overlapping section placed against the outer side of an overlapped section.

According to another embodiment, the invention is characterized in that the absorbent element extends in its longitudinal direction over the whole of the crotch section and slightly up over the front section in the direction of the, during product usage, upper side edge of the second piece.

According to one embodiment, the product according to the invention is characterized in that the said elastic member is constituted by an elastic non-woven or an elastic film.

According to another embodiment, the product according to the invention is characterized in the the said elastic member is constituted by elastic tape or threads.

According to another embodiment, the invention is characterized in that the absorbent product is constituted by a two-piece product having an absorbent element in the form of a detachable insert which is intended to be fitted inside pants incorporated in the product, in that the insert has a rear liquid-tight layer provided with fastening members by means of which the insert is detachably anchored in the pants, and in that the insert, when the front section of the product is pulled down, is arranged to be held in place relative to the pants by means of the said fastening members.

According to a further embodiment, the product according to the invention is characterized by a number of separate absorption bodies which are fitted adjacent to one another in the lateral direction and extend in the longitudinal direction of the product in the direction from the front section to the rear section and which are separable in the lateral direction and longitudinal direction counter to the action of transverse elastic, whereby the separate absorption bodies can be mutually displaced when the front section of the product is pulled down.

### BRIEF DESCRIPTION OF DRAWINGS

The invention will be described in greater detail below with reference to illustrative embodiments shown in the appended drawings, in which:
- Figure 1: shows diagrammatically a phase in the development of an absorbent-product according to a-first embodiment.
- Figure 2: shows diagrammatically in flat form an assembled product according to the first embodiment.
- Figure 3: shows in perspective the product according to Figure 2.
- Figure 4: shows a section along the line IV-iv in Figure 1.
- Figure 5: shows diagrammatically in flat form an example of an absorbent element incorporated in an absorbent product according to the invention.
- Figure 6: shows diagrammatically in flat form another example of an absorbent element incorporated in an absorbent product according to the invention.
- Figure 7: shows diagrammatically a phase in the development of an absorbent product according to a second embodiment.
- Figure 8: shows in flat form an assembled product according to the second embodiment.
- Figure 9: shows a longitudinal section through a further example of an absorbent element for use in an absorbent product according to the invention.
- Figure 10: shows in plan view another example of an absorbent element for use in an absorbent product according to the invention.
- Figure 11: shows a broken section through an example of a deformation zone in an absorbent element in an absorbent product according to the invention.
- Figure 12: shows in plan view an insert incorporated in a two-piece product according to the invention.
- Figure 13: shows a further embodiment of an absorbent product according to the invention.
- Figure 14: shows diagrammatically another illustrative embodiment of an absorbent product according to the invention.
- Figure 15: shows diagrammatically a somewhat modified embodiment in relation to the embodiment according to Figure 1.

### MODE(S) FOR CARRYING OUT THE INVENTION

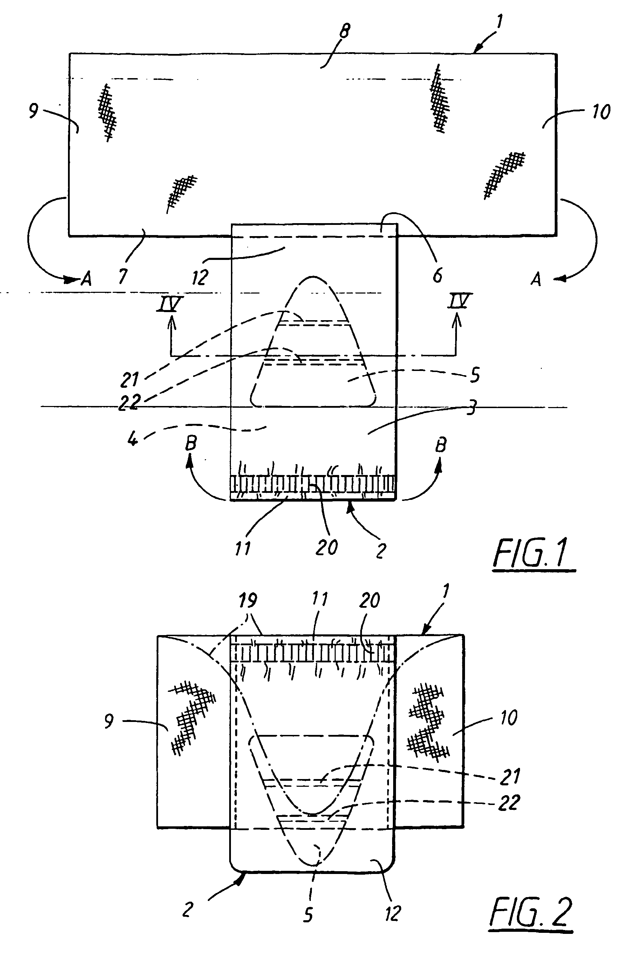

As can be seen from Figure 1, the product in the embodiment according to Figure 1 comprises a first piece 1. This is elastically stretchable and is shown in figure in flat and evenly extended state, in which the elastic piece 1 is essentially rectangular. The elastic first piece 1 can be formed from conventional materials familiar to the person skilled in the art, such as woven elastic materials, elastic non-wovens or elastic films. The essential point is that the elastic piece is stretchable for a user when putting on the product and that it fits tight with suitable tightening around the user when the article is worn. The tightening is adjusted, of course, by means of size and elastic stretchability.

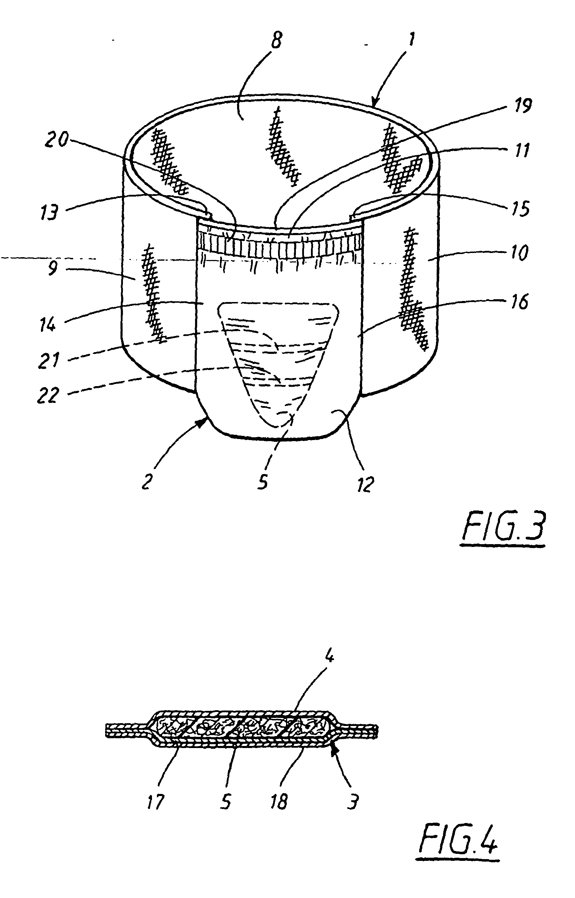

The product incorporates a second piece 2, which, as can be seen from Figures 1 and 4, comprises an outer layer 3, an inner layer 4 and an absorbent element 5 fitted between these. In the embodiment shown in Figure 1, this is in flat form essentially triangular. The choice of material in the absorption element is not critical but can be chosen from amongst materials or material combinations familiar to the person skilled in the art. For example, the absorbent element can be made up of cellulose fluff with superabsorbent material in powder or fibre form mixed in with this. The outer layer 3 can consist, for example, of a polyethylene film of a type conventionally used for absorbent products. A liquid-tight film in combination with an outer fibre layer is suitable if an absorbent product having a more textile-like appearance is sought. The inner layer 4 can be formed from a liquid-permeable non-woven material. The second piece 2 is elongated and is fitted with its longitudinal direction perpendicular to the longitudinal direction of the first piece 1. The second piece 2 is connected by an end section 6 to the one longitudinal edge section 7 of the first piece 1 and centrally on this. The connection can be realized, for example, by the use of bonding agent, heat bonding or ultrasonic bonding.

In Figure 1, the arrows A and B are used to-illustrate how the first and second pieces are folded for the formation of the pants-shaped absorbent product shown in Figures 2 and 3. The elastic piece 1 is folded in according to the shown arrows A to form the rear section 8 and side sections 9, 10 of the products, whereas the second piece is upwards according to the arrows B to form the front section 11 and crotch section 12 of the product.

As can clearly be seen from Figure 3, the one end section 13 of the first piece is fitted in slightly overlapping arrangement with a first side edge section 14 of the second piece and the second end section 15 of the first piece is correspondingly fitted in slightly overlapping arrangement with a second side edge section 16 of the second piece. These overlapping parts are mutually connected, for example by means of bonding agent, heat bonding or ultrasonic bonding. Alternatively, the front section and the crotch section can be detachably fitted along the said overlapping sections 13, 14 and 15, 16 respectively. A detachable connection of this kind can be constituted, for example, by a hook member (not shown), whereby the pants-like product can be opened and subsequently closed up again with the same fit and tightening.

In Figure 4, the thickness of the second piece has been exaggerated for the sake of clarity. In the figure, an outer layer 3 consisting of a laminate of a plastics film 17 and an outermost non-woven layer 18 is shown. The inner layer 4 can be constituted by a liquid-permeable non-woven of a type familiar to the person skilled in the art.

The front section 11 is provided, up to close to its upper limit edge 19, with an elastic member 20 in the form of an elastic tape. This is fitted pretensioned on any of the layers 3, 4 and 5 incorporated in the front section. The elastic member, over the whole of its working range, expediently has an essentially constant tensile stress when stretched, which tensile stress is expediently in the same order of magnitude as the tensile stress in the elastic first piece 1. An upper section of the -elastic first piece 1 forms, together with the elastic member 20, a circumferential elastic waist region.

The elastic member 20 expediently has higher elastic stretchability than the elastic first piece which means that the elastic member has greater elastic travel than the elastic first piece upon the expansion of the elastic waist region. The aim of this difference in elastic travel when subjected to the same tensile force is that the front section of the pants-like product will be able to be pulled down without risk of the product being pulled down, in its entirety, from the waist region of the user. During use, the front section of the pants will be able to be pulled down in front counter to the action of the said elastic member 20 to a position in which the upper limit edge 19 of the front section in the middle region of the front section is situated below the penis of the user. The aim is that the user, when visiting a urinal, without any problem whatsoever and with one hand, will be able to pull down just the front section to the said position, substantially corresponding to that indicated by dashed lines in Figure 2, in which the dashed lines 19 are the upper limit edge of the front section after this has been pulled down. This will be able to be effected without the pants being pulled down in their entirety. In the embodiment shown in Figures 1-4, the hip sections of the product on the side pieces 9 and 10, as can be seen from Figure 2, when the front section of the product is pulled down, will essentially be unaffected and will remain in fitted position on the hip bone of the user.

In order that the pants-like product will be able to be pulled down at the front, as intended, in order to bare the penis of the user, it is also required that the absorbent element 5 is configured such that it can be displaced or deformed in a controlled manner without it becoming damaged.

The absorbent element 5, so as not to give rise to irritating chafing in the crotch of the user, has been configured such that it tapers in the direction pointing rearwards during product usage. A complication with this tapered configuration is that the absorbent element is at its narrowest in the section which, during product usage, is situated substantially directly in front of the genitals of the user, in which section the absorbency requirement is greatest. In order, as far as practicable, to avoid the risk of leakage in the narrower section of the absorbent element according to one embodiment in which superabsorbent material in particle or fibre form is incorporated in the absorbent element, it can be expedient for the superabsorbent material to be applied in increasing concentration in the said direction of taper. A shape of the absorbent element 5 which tapers in the direction of the crotch section is also more discrete than, for example, a rectangular absorption body which is folded in an uncontrolled manner in the crotch and is more bulky, whilst, at the same time, formed folds can conduct received urine in the lateral direction out of the absorbent product and cause urine leakage.

In the illustrative embodiment shown in Figures 1-4, the absorbent element is provided with deformable zones in the form of fold notches 21, 22, along which the absorbent element is bent when the front section of the product is pulled down. The absorbent element 5 is firstly bent at the fold notch 21 and thereafter, as the product continues to be pulled down, at the fold notch 22. At least the upper fold notch 21 is configured such that the absorbent element here bends out from the user. Once the front section of the product has been fully pulled down, the absorbent element assumes a temporary Z-like shape in longitudinal section. When the user has fulfilled his needs and lets go of the pants and the elastic member 20, the front section of the pants and the absorbent element 5 return to their original state of use through the action of the elastic in the elastic member 20.

In the embodiment shown in Figures 1-4, the deformation zones have the form of transverse fold notches. This allows the product to be successfully pulled down and returned to its original position without residual deformation. If a different down-fold is desired, during which the absorbent element, in the course of being folded down, requires less space out from the user, the fold notches can be arranged with different extent, for example such that the fold notches acquire a U-like or V-like shape with the arms of the said U or V-shape essentially parallel with the side edges of the downward-tapered absorbent element.

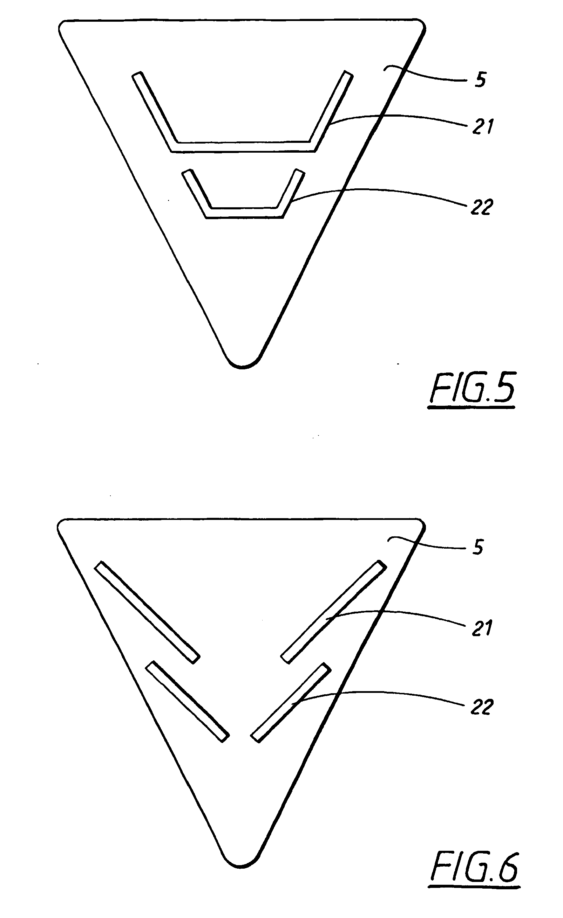

In Figures 5 and 6, two examples are shown of U-shaped or V-shaped fold notches 21, 22 arranged on an absorbent element 5. The number of fold notches can, of course, be more than two in the vertical direction, depending on how the absorption body is wished to be deformed when the front section is pulled down.

The fold notches can also be in the form of relatively wide recesses, arranged, otherwise, with the same extent as shown in Figures 1-6.

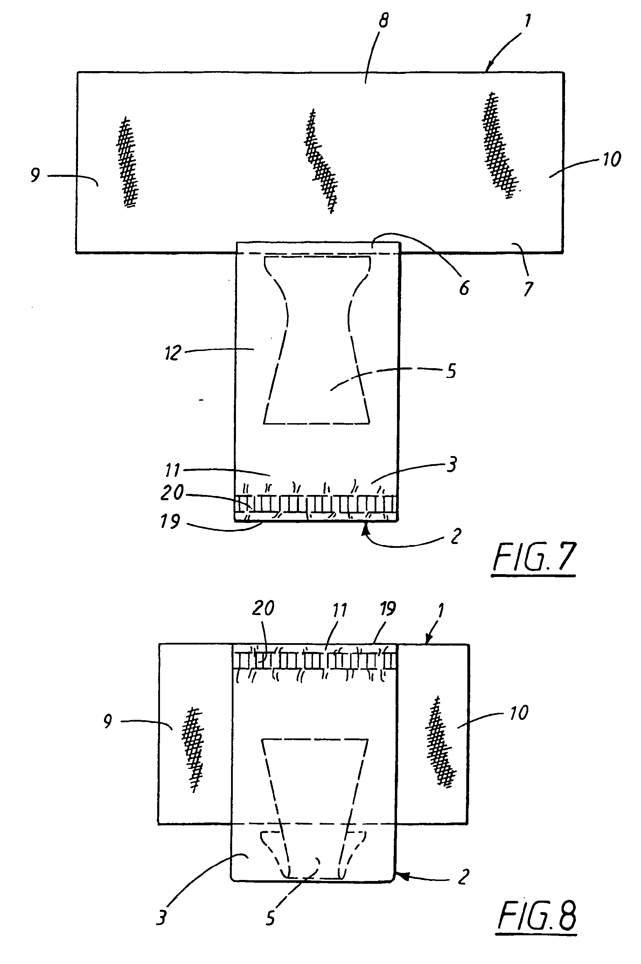

In the embodiments according to Figures 1-6, the absorbent element 5 is essentially triangular in shape and, during product usage, is situated wholly on the user in front and does not cover the whole of the crotch section of the user. The absorbent element can, of course, have a different shape, for example can be essentially hourglass-shaped as shown in Figures 7 and 8. In Figures 7 and 8, the same reference notations have been used compared to similar components in the embodiments according to Figures 1-4.

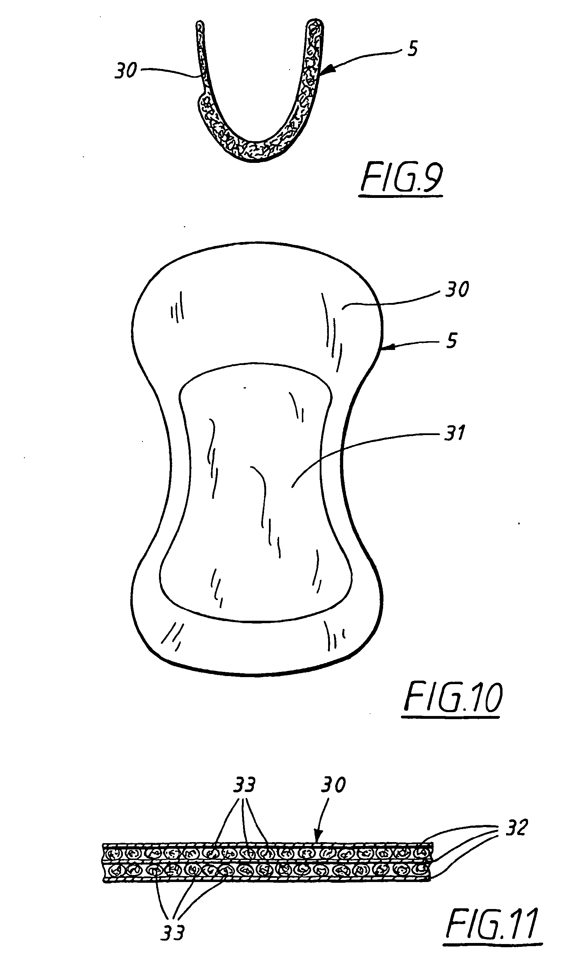

In Figure 9, an alternative realization of an absorbent element 5 is shown, having a deformable zone 30. The absorbent element is shown diagrammatically in cross section in an imaginary usage position. Other details in the absorbent product have not been included, for the sake of simplicity. The deformable zone has here been produced by virtue of it being substantially thinner and having lower bending resistance than the rest of the absorbent element.

In Figure 10 a similar illustrative embodiment of an absorbent element 5 having a deformable zone 30 is shown in flat form. The absorbent element here comprises two separate absorption bodies fitted one on top of the other, the one with greater extent being thinner and having substantially lower bending resistance than the two absorption bodies together.

An illustrative embodiment of an absorption body serving as a deformation zone is shown in Figure 11 in diagrammatic representation and on enlarged scale in the form of a part-section through the absorption body. The absorption body, which has here been denoted by 30, comprises a number of material layers 32 of non-woven material or tissue with a bed of highly absorbent particles 33 between neighbouring material layers. An absorption body of this type, compared with other conventional absorption bodies, for example made of compressed fluff substance, has little or no inherent stiffness and can therefore be easily deformed in connection with a front section of an absorbent product according to the invention being pulled down. It is expedient for the highly absorbent particles to be bound to the material layers to prevent the absorption body from being permanently deformed as a result of displacement and agglomeration of the particles when the front section of the absorbent product is pulled down by the user. This bonding will, of course, not be such that the absorption body is substantially stiffened compared with when the particles are totally unbound.

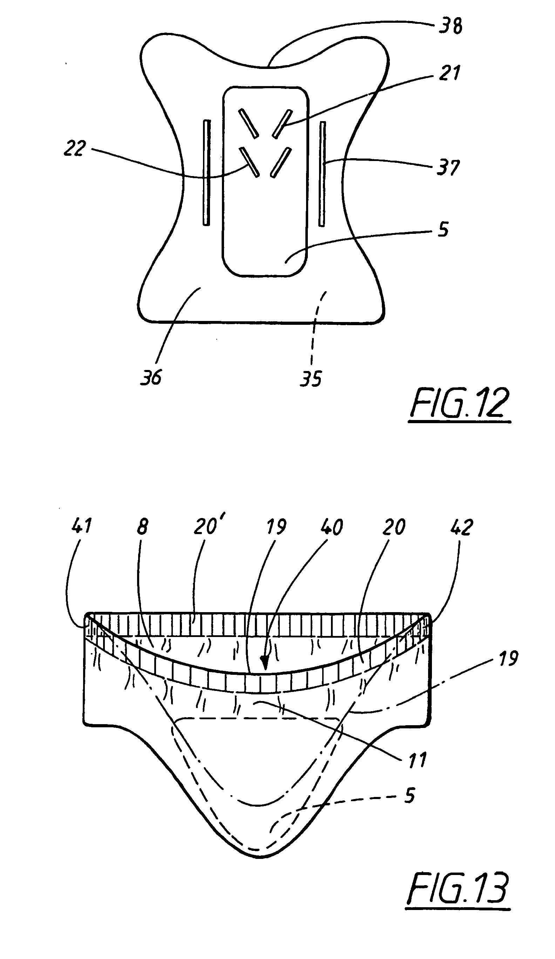

The absorbent product according to the invention can be constituted by a two-piece product having an absorbent element in the form of a detachable insert, which is intended to be fitted inside pants incorporated in the product. An illustrative embodiment of an insert is shown in Figure 12. The insert incorporates an absorbent element 5, which is provided with essentially V-shaped deformation zones 21, 22. The absorbent element is enclosed in a sheath comprising a liquid-tight layer 35 on that side which, in use, faces away from the user and a liquid-permeable layer 36 on the opposite side. The two layers extend in the lateral direction beyond the absorbent element and are there mutually- joined. In the shown illustrative embodiment, the insert is provided with longitudinal edge elastic 37 for the formation of leakage protection in the lateral direction of the product. For the marking of the end which is to be fitted facing the front, the shown insert containing the layers has a recess 38 in this end. The insert is intended to be fitted in outer pants (not shown), provided with an elastic member of the type described in connection with the embodiments according to Figures 1-4. The liquid-tight layer is expediently provided on its outer side with one or more fastening member(s) (not shown) for detachable anchorage to the outer pants, so that the insert sits fixedly at the intended place inside the pants during product usage and when the pants and the deformable section of the product are pulled down by the user during a toilet visit.

In the illustrative embodiment of an absorbent product according to the invention shown in Figure 13, the front section of the product has at the waist region a - viewed from the crotch region - concave recess 40 between the hip sections, here denoted by 41, 42. During product usage, the front section of the product, as can be seen from Figure 13, hence acquires a low pants cut. Compared with a straight cut, this reduces the amount by which the upper limit edge 19 of the product in the middle region of the front section needs to be pulled down in order to bring the said limit edge into position below the penis of the user. The absorbent product can, for example, be provided at the said hip sections 41, 42 with friction members (not shown) in the form of friction glue or a foam material with high surface friction on the inner or outer side of the product. The friction members are intended to help the product remain wholly in place when the user pulls down the front section of the product in order to free his penis in connection with a toilet visit. In the embodiment according to Figure 13, parts corresponding to similar components in other embodiments have been provided with the same reference notations. The upper limit edge 19 of the front section, after the latter has been pulled down, has been marked in Figure 13 with a dash-dot line 19. The rear section 8 of the absorbent product is provided with waist elastic 20'.

The product according to the invention has a pants-like shape. The pants can be wholly or partially elastic with the same or different elastic properties in different regions. The elastic can be constituted by elastic non-woven material, elastic films or a combination of these materials. The elastic can also be produced by elastic threads or tape, which are fitted with the same or different pretensioning in different regions. The product expediently has a circumferential elastic waist region, which forms waist elastic for keeping the product in place on the user. The elastic member 20 on the front section of the product can constitute a section of the elastic waist region and can have the same or different elastic properties compared with the rest of the waist elastic. The essential point is that the elastic member allows the front section of the product to be able to be pulled down without difficulty so that the upper limit edge of the product in the middle region of the front section is situated below the penis of the user.

Elastic film and elastic non-woven material can be configured such that it is elastic in more than one direction. Materials of this type can, of course, be used in the front section of the product over the whole or sections of this. Like elastic threads or elastic tape, films or non-wovens which are elastic in only one direction can also be used to form the elastic member.

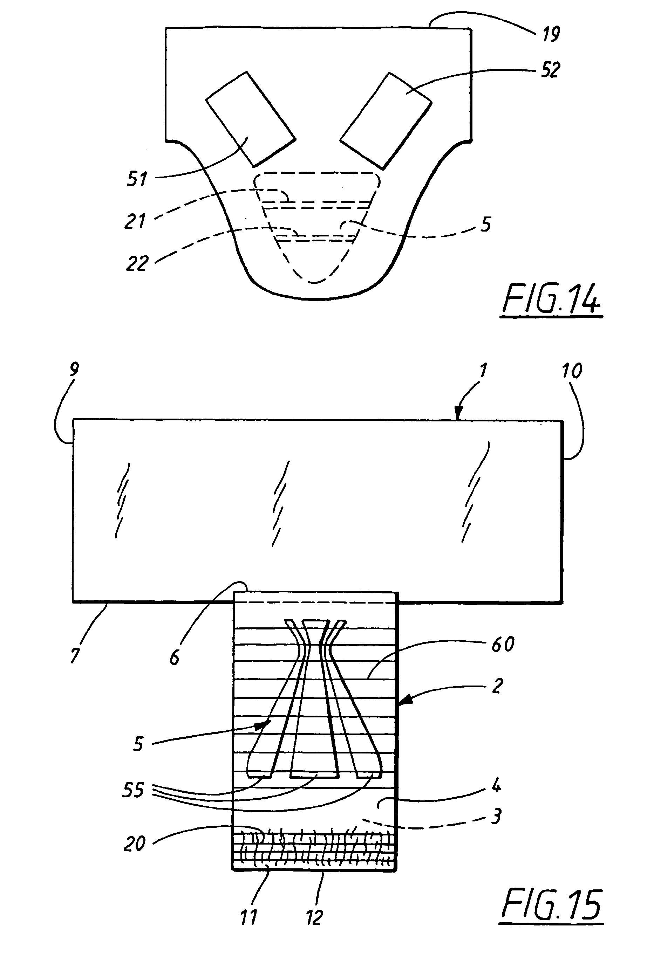

In Figure 14, an illustrative embodiment of a product according to the invention is shown diagrammatically, having an elastic member formed by two zones 51, 52 of a film which is elastic in the one direction in the said zones. The direction of the elastic in the said zones is arranged such that this is effective in the longitudinal direction of the said zones. When the front section of the product is pulled down, the intended pulling down of the elastically stretchable zones is facilitated.

In the embodiment shown in Figure 15, those parts corresponding to similar components in the embodiment according to Figure 1 have been provided with the same reference numerals. The absorbent element 5 according to this embodiment is constituted by a number of separate absorption bodies 55 which are fitted adjacent to one another in the lateral direction and extend in the longitudinal direction of the product from the front section to the rear section and which are separable in the vertical direction and the lateral direction counter to the action of transverse elastic 60. The separate absorption bodies can hence be mutually displaced when the front section of the product is pulled down. The absorbent element is in this case configured correspondingly to the description given in WO 91/09580, to which allusion is made as a reference. The elastic member 20 has here been formed by a number of parallel elastic threads.

The invention is not limited to the illustrative embodiments described above, but rather a number of modifications are possible within the scope of the following patent claims.

## Claims

1. Pants-shaped absorbent product, such as incontinence pants, intended for males, comprising a front section (11), a rear section (8), a crotch section (12), side sections (9, 10), which are intended to connect the front section and the rear section in the lateral direction and which comprise hip sections, an elastic waist region and an absorbent element (5) which, when the article is in use, is intended to at least cover the penis of the user, and further comprising a liquid-tight outer layer (3), which is intended to cover the absorbent element at least on the side thereof facing away from the user when the product is worn, **characterized in that** the front section has at least one elastic member (20), which, during product usage, enables the front section (11) of the product to be pulled down, counter to the action of the said elastic member (20), to a position in which the upper limit edge (19) of the front section in the middle region of the front section is situated below the penis of the user, at the same time as at least the hip sections of the product are arranged to be held in place around the waist of the user, **in that** the absorbent element (5) is configured with one or more deformation zones (21, 22), which enable those parts of the absorbent element (5) which are situated above and over the penis of the user during product usage to be drawn down together with the rest of the front section when the front section (11) of the product is pulled down, and **in that** the front section of the product, together with the absorbent element (5), are arranged to be returned by the elastic member (20) to their original usage position.

2. Absorbent product according to Claim 1, **characterized in that** the said elastic member (20), over the whole of its working range, has an essentially constant tensile stress when stretched, which tensile stress is in the same order of magnitude as the tensile stress in the elastic waist region.

3. Absorbent product according to Claim 1 or 2, **characterized in that** the elastic member (20) is constituted by a section of the elastic waist region.

4. Absorbent product according to Claim 3, **characterized in that** the said section extends over the front section (11) between the said hip sections.

5. Absorbent product according to Claim 4, **characterized in that** the elastic waist region extends over the whole of the front section (11), rear section (8) and side sections (9, 10) of the product and **in that** the said section of the elastic waist region which serves as the elastic member (20) has higher elastic stretchability than the rest of the elastic waist region.

6. Absorbent product according to any of the preceding claims, **characterized in that** the absorbent element (5) is of thinner (30) and more flexible configuration in the deformable zone compared with the rest of the absorbent element (5).

7. Absorbent product according to any of Claims 1-5, **characterized in that** the absorbent element (5) incorporates deformable zones (21, 22) in the form of fold notches for the absorbent element (5), along which fold notches the product is bent when the front section (11) of the product is pulled down.

8. Absorbent product according to Claim 7, **characterized in that** the said fold notches are constituted by recesses, through-slots or compression lines in the absorbent element.

9. Absorbent product according to any of Claims 1-6, **characterized in that** the absorbent element (5) is constituted by at least two absorption bodies (30, 31) of different extent, **in that** only one of these (30) extends into the deformation zone and **in that** the absorption body (5) extending into the deformation zone has little or no inherent stiffness.

10. Absorbent product according to any of the preceding claims, **characterized in that** the front section of the product (11), at the waist region, has a, viewed from the crotch region, concave recess (40) between the said hip sections (41, 42), whereby the front section of the product, during product usage, acquires a low pants cut, which, compared with a straight cut, reduces the amount by which the upper limit edge (19) of the product in the middle region of the front section (11) needs to be pulled down in order to bring the said limit edge into position below the penis of the user.

11. Absorbent product according to any of the preceding claims, **characterized in that** the hip sections of the product are provided with friction members, which are arranged to help the product as a whole to be held in place on the user as the front section of the product is pulled down.

12. Absorbent product according to any of the preceding claims, **characterized in that** the said elastic waist region, at least in part, is formed from an elastic first piece (1) which, in the extended state, is essentially rectangular and which is intended to partially surround the trunk of the user and form the rear section (8) and side sections (9, 10), comprising hip sections (41, 42), of the pants-like product, **in that** a second piece (2), incorporated in the product, is configured to form the front section (11) and crotch section (12) of the pants-like product, **in that** the said second piece (2) is elongated with two opposing end edges and two opposing longitudinal edges, **in that** the width of the second piece (2), at least at the crotch section (12), is less than the length of the first piece (1), **in that** the second piece (2) with its longitudinal direction is arranged perpendicularly to the longitudinal direction of the first piece (1) and is connected by a first end section (6) to the one longitudinal edge section (7) of the first piece (1) and centrally on this, **in that** the one end section (13) of the first piece (1) is connected to a first side edge section (14) of the second piece (2), and **in that** the second end section (15) of the first piece is correspondingly connected to a second side edge section (16) of the second piece (2), and the absorbent element (5) is fitted, in its entirety, on the second piece (2).

13. Absorbent product according to Claim 12, **characterized in that** the said connected side edge sections (14, 16) and end sections (13, 15) of the said first (1) and second pieces (2), prior to connection, are fitted in overlapping arrangement with the inner side of an overlapping section placed against the outer side of an overlapped section.

14. Absorbent product according to any of the preceding claims, **characterized in that** the absorbent element (5) extends in its longitudinal direction over the whole of the crotch section (12) and slightly up over the front section (11) in the direction of the, during product usage, upper side edge of the second piece.

15. Absorbent product according to any of the preceding claims, **characterized in that** the said elastic member (20) is constituted by an elastic non-woven or an elastic film.

16. Absorbent product according to any of Claims 1-15, **characterized in that** the said elastic member (20) is constituted by elastic tape or threads.

17. Absorbent product according to any of the preceding claims, **characterized in that** the absorbent product is constituted by a two-piece product having an absorbent element (5) in the form of a detachable insert which is intended to be fitted inside pants incorporated in the product, **in that** the insert has a rear liquid-tight layer (35) provided with fastening members by means of which the insert is detachably anchored in the pants, and **in that** the insert, when the front section of the product is pulled down, is arranged to be held in place relative to the pants by means of the said fastening members.

18. Absorbent product according to Claim 1, **characterized in that** the absorbent element (5) is constituted by a number of separate absorption bodies (55) which are fitted adjacent to one another in the lateral direction and extend in the longitudinal direction of the product in the direction from the front section (11) to the rear section (8) and which are separable in the lateral direction and longitudinal direction counter to the action of transverse elastic (60), whereby the separate absorption bodies can be mutually displaced when the front section of the product is pulled down.

## Patentansprüche

1. Hosenförmiges Absorptionsprodukt, wie etwa Inkontinenzhosen, gedacht für männliche Personen, umfassend einen Frontabschnitt (11), einen Rückabschnitt (8), einen Schrittabschnitt (12), Seitenabschnitte (9, 10), die dazu gedacht sind, den Frontabschnitt und den Rückabschnitt in der seitlichen Richtung zu verbinden und die Hüftabschnitte umfassen, einen elastischen Taillenbereich und ein Absorptionselement (5), das, wenn der Artikel in Benutzung ist, dazu gedacht ist, zumindest den Penis des Benutzers zu bedecken, und ferner umfassend eine flüssigkeitsdichte Außenlage (3), die dazu gedacht ist, das Absorptionselement zumindest an seiner Seite, die dem Benutzer abgeneigt ist, wenn das Produkt getragen wird, zu bedecken, **dadurch gekennzeichnet, dass** der Frontabschnitt zumindest ein elastisches Element (20) aufweist, das während der Produktbenutzung den Frontabschnitt (11) des Produkts dazu in die Lage versetzt, gegen die Wirkung des elastischen Elements (20) in eine Position heruntergezogen zu werden, in der die obere Randkante (19) des Frontabschnitts im mittleren Bereich des Frontabschnitts unterhalb des Penis des Benutzers angeordnet ist, zur gleichen Zeit wie zumindest die Hüftabschnitte des Produkts so angeordnet sind, dass sie an ihrem Ort um die Hüfte des Benutzers herum gehalten werden, dass das Absorptionselement (5) mit einer oder mehreren deformierbaren Zonen (21, 22) ausgestaltet ist, die diejenigen Teile des Absorptionselements (5), die oberhalb und über dem Penis des Benutzers während der Produktbenutzung angeordnet sind, in die Lage versetzen, zusammen mit dem Rest des Frontabschnitts heruntergezogen zu werden, wenn der Frontabschnitt (11) des Produkts heruntergezogen wird, und dass der Fontabschnitt des Produkts zusammen mit dem Absorptionselement (5) so angeordnet ist, dass sie durch das elastische Element (20) in ihre ursprüngliche Benutzungsposition zurückkehren.

2. Absorptionsprodukt nach Anspruch 1, **dadurch gekennzeichnet, dass** das elastische Element (20) über seinen gesamten Arbeitsbereich eine im Wesentlichen konstante Zugspannung aufweist, wenn es gedehnt wird, welche Zugspannung in derselben Größenordnung wie die Zugspannung im elastischen Taillenbereich ist.

3. Absorptionsprodukt nach Anspruch 1 oder 2, **dadurch gekennzeichnet, dass** das elastische Element (20) durch einen Abschnitt des elastischen Taillenbereichs gebildet wird.

4. Absorptionsprodukt nach Anspruch 3, **dadurch gekennzeichnet, dass** sich der Bereich über den Frontabschnitt (11) zwischen die Hüftabschnitte erstreckt.

5. Absorptionsprodukt nach Anspruch 4, **dadurch gekennzeichnet, dass** sich der elastische Taillenbereich über den ganzen Frontabschnitt (11), Rückabschnitt (8) und die Seitenabschnitte (9, 10) des Produkts erstreckt und dass der Abschnitt des elastischen Taillenbereichs, der als das elastische Element (20) dient, eine höhere elastische Dehnbarkeit als der Rest des elastischen Taillenbereichs aufweist.

6. Absorptionsprodukt nach einem der vorhergehenden Ansprüche, **dadurch gekennzeichnet, dass** das Absorptionselement (5) eine dünnere (30) und flexiblere Ausgestaltung in der deformierbaren Zone, verglichen mit dem Rest des Absorptionselements (5), aufweist.

7. Absorptionsprodukt nach einem der Ansprüche 1 bis 5, **dadurch gekennzeichnet, dass** das Absorptionselement (5) deformierbare Zonen (21, 22) in Form von gefalteten Einschnitten für das Absorptionselement (5) enthält, entlang welcher gefalteten Einschnitte das Produkt gebogen wird, wenn der Frontabschnitt (11) des Produkts heruntergezogen wird.

8. Absorptionsprodukt nach Anspruch 7, **dadurch gekennzeichnet, dass** die gefalteten Einschnitte durch Ausnehmungen, Durchgangsschlitze oder Kompressionslinien in dem Absorptionselement gebildet werden.

9. Absorptionsprodukt nach einem der Ansprüche 1 bis 6, **dadurch gekennzeichnet, dass** das Absorptionselement (5) durch zumindest zwei Absorptionskörper (30, 31) verschiedener Ausdehnung gebildet wird, dass sich nur einer von ihnen (30) in die deformierbare Zone erstreckt und dass sich der Absorptionskörper (5), der sich in die deformierbare Zone erstreckt, wenig oder keine inhärente Steifigkeit aufweist.

10. Absorptionsprodukt nach einem der vorhergehenden Ansprüche, **dadurch gekennzeichnet, dass** der Frontabschnitt des Produkts (11) im Taillenbereich eine vom Schrittabschnitt aus gesehen konkave Ausnehmung (40) zwischen den Hüftabschnitten (41, 42) aufweist, wodurch der Frontabschnitt des Produkts während der Produktbenutzung einen niedrigen Hosenschnitt annimmt, der im Vergleich mit einem geraden Schnitt das Maß, um das die obere Randkante (19) des Produkts im Mittelbereich des Frontabschnitts (11) heruntergezogen werden muss, um die Randkante in ihre Position unterhalb des Penis des Benutzers zu bringen, reduziert.

11. Absorptionsprodukt nach einem der vorhergehenden Ansprüche, **dadurch gekennzeichnet, dass** die Hüftabschnitte des Produkts mit Reibungselementen versehen sind, die dazu angeordnet sind, dabei zu helfen, dass das Produkt insgesamt am Benutzer gehalten wird, wenn der Frontabschnitt des Produkts heruntergezogen wird.

12. Absorptionsprodukt nach einem der vorhergehenden Ansprüche, **dadurch gekennzeichnet, dass** der elastische Hüftbereich zumindest teilweise aus einem elastischen ersten Stück (1) gebildet ist, das im ausgedehnten Zustand im wesentlichen rechteckig ist und das dazu gedacht ist, teilweise den Rumpf des Benutzers zu umgeben und den Rückabschnitt (8) und Seitenabschnitte (9, 10) mit Hüftabschnitten (41, 42) des hosenähnlichen Produkts zu bilden, dass ein zweites Stück (2), das in dem Produkt enthalten ist, dazu ausgestaltet ist, den Frontabschnitt (11) und Schrittabschnitt (12) des hosenähnlichen Produkts zu bilden, dass das zweite Stück (2) mit zwei einander gegenüberliegenden Endkanten und zwei einander gegenüberliegenden Längskanten verlängert ist, dass die Breite des zweiten Stücks (2) zumindest im Schrittabschnitt (12) kleiner als die Länge des ersten Stücks (1) ist, dass das zweite Stück (2) mit seiner Längsrichtung senkrecht zu der Längsrichtung des ersten Stücks (1) angeordnet und durch einen ersten Endabschnitt (6) mit dem einen Längskantenabschnitt (7) des ersten Stücks (1) und zentral daran verbunden ist, dass der eine Endabschnitt (13) des ersten Stücks (1) mit einem ersten Seitenkantenabschnitt (14) des zweiten Stücks (2) verbunden ist und dass der zweite Endabschnitt (15) des ersten Stücks entsprechend an einem zweiten Seitenkantenabschnitt (16) des zweiten Stücks (2) verbunden ist und das Absorptionselement (5) vollständig an dem zweiten Stück (2) angebracht ist.

13. Absorptionsprodukt nach Anspruch 12, **dadurch gekennzeichnet, dass** die verbundenen Seitenkantenabschnitte (14, 16) und Endabschnitte (13, 15) des ersten (1) und zweiten Stücks (2) vor der Verbindung in überlappender Anordnung befestigt sind, wobei die Innenseite eines überlappenden Abschnitts gegen die Außenseite eines überlappten Abschnitts platziert ist.

14. Absorptionsprodukt nach einem der vorhergehenden Ansprüche, **dadurch gekennzeichnet, dass** sich das Absorptionselement (5) in seiner Längsrichtung über den gesamten Schrittabschnitt (12) und etwas herauf über den Frontabschnitt (11) in der Richtung der bei Produktbenutzung oberen Seitenkante des zweiten Stücks erstreckt.

15. Absorptionsprodukt nach einem der vorhergehenden Ansprüche, **dadurch gekennzeichnet, dass** das elastische Element (20) durch ein elastisches Vlies oder eine elastische Folie gebildet ist.

16. Absorptionsprodukt nach einem der Ansprüche 1 bis 15, **dadurch gekennzeichnet, dass** das elastische Element (20) durch elastisches Band oder elastische Fäden gebildet ist.

17. Absorptionsprodukt nach einem der vorhergehenden Ansprüche, **dadurch gekennzeichnet, dass** das Absorptionsprodukt durch ein zweiteiliges Produkt mit einem Absorptionselement (5) in Form einer abnehmbaren Einlage gebildet ist, die dazu gedacht ist, innerhalb von Hosen, die in dem Produkt eingearbeitet sind, befestigt zu werden, dass die Einlage eine rückseitige flüssigkeitsdichte Lage (35) aufweist, die mit Befestigungselementen versehen ist, durch die die Einlage abnehmbar in den Hosen verankert ist, und dass die Einlage, wenn der Frontabschnitt des Produkts heruntergezogen wird, dazu angeordnet ist, in Bezug auf die Hosen durch die Befestigungselemente gehalten zu werden.

18. Absorptionsprodukt nach Anspruch 1, **dadurch gekennzeichnet, dass** das Absorptionselement (5) durch mehrere separate Absorptionskörper (55) gebildet wird, die nebeneinander in seitlicher Richtung befestigt sind und sich in der Längsrichtung des Produkts in der Richtung von dem Frontabschnitt (11) zu dem Rückabschnitt (8) erstrecken und die in der seitlichen Richtung und der Längsrichtung gegen die Wirkung einer Querelastizität (60) trennbar sind, wodurch die separaten Absorptionskörper gegeneinander verschoben werden können, wenn der Frontabschnitt des Produkts heruntergezogen ist.

## Revendications

1. Article absorbant en forme de culotte, tel qu'une culotte pour incontinence, destiné aux hommes, comprenant une zone avant (11), une zone arrière (8), une zone d'entrejambe (12), des zones latérales (9, 10) qui sont destinées à relier la zone avant et la zone arrière dans la direction latérale et qui comprennent des zones de hanches, une zone élastique pour la taille et un élément absorbant (5) qui, lorsque l'article est en cours d'utilisation, est destiné à recouvrir au moins le pénis de l'utilisateur et comprenant en outre une couche extérieure (3) imperméable aux liquides qui est destinée à recouvrir l'élément absorbant au moins sur le côté de celui-ci qui est orienté à l'écart de l'utilisateur lorsque l'article est porté, **caractérisé en ce que** la zone avant comporte au moins un élément élastique (20) qui, lors de l'utilisation de l'article, permet que la zone avant (11) de l'article soit tirée vers le bas, à l'encontre de l'action dudit élément élastique (20), jusqu'à atteindre une position dans laquelle le bord supérieur formant la limite (19) de la zone avant dans la région médiane de la zone avant est situé sous le pénis de l'utilisateur en même temps qu'au moins les zones de hanches de l'article sont agencées de manière à être maintenues en place autour de la taille de l'utilisateur, **en ce que** l'élément absorbant (5) a une configuration comportant une ou plusieurs zones de déformation (21, 22) qui permettent que les parties de l'élément absorbant (5) qui sont situées au-dessus du pénis de l'utilisateur et sur celui-ci lors de l'utilisation de l'article soient tirées vers le bas avec le reste de la zone avant lorsque la zone avant (11) de l'article est tirée vers le bas, et **en ce que** la zone avant de l'article, en même temps que l'élément absorbant (5) sont agencés de manière à être ramenés par l'élément élastique (20) jusqu'à leur position d'utilisation d'origine.

2. Article absorbant selon la revendication 1, **caractérisé en ce que** ledit élément élastique (20) subit une contrainte de traction essentiellement constante sur toute sa plage de travail, laquelle contrainte de traction a une ampleur du même ordre que la contrainte de traction dans la zone de taille élastique.

3. Article absorbant selon la revendication 1 ou 2, **caractérisé en ce que** l'élément élastique (20) est constitué d'une partie de la zone de taille élastique.

4. Article absorbant selon la revendication 3, **caractérisé en ce que** ladite partie s'étend sur la zone avant (11) entre lesdites zones de hanches.

5. Article absorbant selon la revendication 4, **caractérisé en ce que** la zone de taille élastique s'étend sur la totalité de la zone avant (11), de la zone arrière (8) et des zones latérales (9, 10) de l'article et **en ce que** ladite partie de la zone de taille élastique qui sert d'élément élastique (20) a une extensibilité élastique supérieure à celle du reste de la zone de taille élastique.

6. Article absorbant selon l'une quelconque des revendications qui précèdent, **caractérisé en ce que** l'élément absorbant (5) a une configuration plus mince (30) et plus souple dans la zone déformable si on la compare au reste de l'élément absorbant (5).

7. Article absorbant selon l'une quelconque des revendications 1 à 5, **caractérisé en ce que** l'élément absorbant (5) comprend des zones déformables (21, 22) se présentant sous forme de marques de pliage destinées à l'élément absorbant (5), le long desquelles marques de pliage l'article est courbé lorsque la zone avant (11) de l'article est tirée vers le bas.

8. Article absorbant selon la revendication 7, **caractérisé en ce que** lesdites marques de pliage sont constituées de creux, de fentes traversantes ou de lignes de compression dans l'élément absorbant.

9. Article absorbant selon l'une quelconque des revendications 1 à 6, **caractérisé en ce que** l'élément absorbant (5) est constitué d'au moins deux corps d'absorption (30, 31) ayant une étendue différente, **en ce qu'**un seul (30) de ceux-ci s'étend dans la zone de déformation et **en ce que** le corps d'absorption (5) qui s'étend dans la zone de déformation a peu de rigidité inhérente ou pas de rigidité inhérente.

10. Article absorbant selon l'une quelconque des revendications qui précèdent, **caractérisé en ce que** la zone avant de l'article (11), comporte, au niveau de la zone de taille, vu à partir de la zone d'entrejambe, une cavité concave (40) entre lesdites zones de hanches (41, 42) grâce à quoi la zone avant de l'article, prend, lors de l'utilisation de l'article, une forme de culotte à taille basse qui, si on la compare à une forme droite, réduit l'amplitude de la traction vers le bas du bord formant la limite supérieure (19) de l'article dans la région médiane de la zone avant (11), nécessaire pour amener ledit bord limite en place sous le pénis de l'utilisateur.

11. Article absorbant selon l'une quelconque des revendications qui précèdent, **caractérisé en ce que** les zones de hanches de l'article sont munies d'éléments de frottement qui sont agencés de manière à faciliter le maintien en place de l'ensemble de l'article sur l'utilisateur lorsque la zone avant de l'article est tirée vers le bas.

12. Article absorbant selon l'une quelconque des revendications qui précèdent, **caractérisé en ce que** ladite zone de taille élastique est constituée au moins partiellement d'une première partie élastique (1) qui, à l'état tendu, est essentiellement rectangulaire et qui est destinée à entourer partiellement le tronc de l'utilisateur et à constituer la zone arrière (8) ainsi que les zones latérales (9, 10), comprenant les zones de hanches (41, 42) de l'article du genre culotte, **en ce qu'**une deuxième partie (2) incorporée dans l'article, est configurée de manière à former la zone avant (11) et la zone d'entrejambe (12) de l'article du genre culotte, **en ce que** la deuxième partie (2) a une forme oblongue, comportant deux bords d'extrémité opposés et deux bords longitudinaux opposés, **en ce que** la largeur de la deuxième partie (2), au moins au niveau de la zone d'entrejambe (12) est inférieure à la longueur de la première partie (1), **en ce que** la deuxième partie (2) a sa direction longitudinale agencée perpendiculairement à la direction longitudinale de la première partie (1) et est reliée par une première partie d'extrémité (6) à une partie (7) de bord longitudinal de la première partie (1) et de façon centrale sur celle-ci, **en ce que** la partie d'extrémité (13) de la première partie (1) est reliée à une première partie (14) de bord latéral de la deuxième partie (2) et **en ce que** la deuxième partie d'extrémité (15) de la première partie est, de manière correspondante, reliée à une deuxième partie (16) de bord latéral de la deuxième partie (2) et **en ce que** l'élément absorbant (5) est mis en place en totalité sur la deuxième partie (2).

13. Article absorbant selon la revendication 12, **caractérisé en ce que** lesdites parties de bord latéral (14, 16) qui ont été reliées et les parties d'extrémité de ladite première partie (1) et de ladite deuxième partie (2) sont, avant leur connexion, placées en étant chevauchées par la face intérieure d'une partie chevauchante mise en place contre la face extérieure d'une partie chevauchée.

14. Article absorbant selon l'une quelconque des revendications qui précèdent, **caractérisé en ce que** l'élément absorbant (5) s'étend dans sa direction longitudinale sur la totalité de la zone d'entrejambe (12) et légèrement au-dessus de la zone avant (11) dans la direction du bord latéral supérieur de la deuxième partie, lors de l'utilisation de l'article.

15. Article absorbant selon l'une quelconque des revendications qui précèdent, **caractérisé en ce que** ledit élément élastique (20) est constitué d'un non-tissé élastique ou d'un film élastique.

16. Article absorbant selon l'une quelconque des revendications 1 à 15, **caractérisé en ce que** ledit élément élastique (20) est constitué d'un ruban élastique ou de fils élastiques.

17. Article absorbant selon l'une quelconque des revendications qui précèdent, **caractérisé en ce que** l'article absorbant est constitué d'un produit en deux pièces comportant un élément absorbant (5) se présentant sous forme d'un insert détachable qui est destiné à être enfilé dans la culotte incorporée dans l'article, **en ce que** l'insert comporte une couche arrière imperméable aux liquides (35) munie d'éléments d'attache au moyen desquels l'insert est ancré de façon détachable dans la culotte et **en ce que** l'insert, lorsque la zone avant de l'article est tirée vers le bas, est agencé de manière à être maintenu en place par rapport à la culotte au moyen desdits éléments d'attache.

18. Article absorbant selon la revendication 1, **caractérisé en ce que** l'article absorbant (5) est constitué d'un certain nombre de corps d'absorption séparés (55) qui sont mis en place à proximité les uns des autres dans la direction latérale et qui s'étendent dans la direction longitudinale de l'article entre la zone avant (11) et la zone arrière (8) et qui peuvent être séparés dans la direction latérale et dans la direction longitudinale à l'encontre de l'action de l'élastique transversal (60) grâce à quoi les corps d'absorption séparés peuvent être déplacés les uns par rapport aux autres lorsque la zone avant de l'article est tirée vers le bas.
